# DEMANDE DE BREVET EUROPEEN

(11) **EP 1 110 570 A1**
(43) Date de publication de la demande: **27.06.2001**
(21) Numéro de dépôt: 00810037.2
(22) Date de dépôt: 17.01.2000
(51) Int. Cl.: A61M 5/30

(54) **Dispositif d'injection hypodermique**

(30) Priorité: 23.12.1999 EP 99811206
(71) Demandeur: Neracher, Arnold, CH-1247 Anières (CH)
(72) Inventeur: Neracher, Arnold, CH-1247 Anières (CH)
(74) Mandataire: WILLIAM BLANC & CIE

(57) **Abrégé**

Un dispositif d'injection hypodermique (1) pour l'administration sous-cutanée, sans aiguille, d'un produit liquide à injecter (2) tel qu'un médicament, un vaccin ou une autre composition pharmaceutique, comporte une ampoule contenant ledit produit liquide à injecter et une substance compressible (7) liquide ou solide, et une partie de buse (11) ayant un orifice. Avant l'utilisation, la substance compressible est comprimée à une pression suffisante pour propulser le produit liquide à injecter, pendant l'utilisation, à travers l'orifice de manière à créer un jet de liquide ayant une vitesse suffisante pour traverser la peau (3) d'un patient.

## Description

La présente invention concerne un dispositif d'injection hypodermique de médicaments ou d'autres compositions pharmaceutiques, tels que des vaccins, sous forme liquide. L'invention concerne notamment un dispositif d'injection sans aiguille.

Il existe de nombreuses publications décrivant des dispositifs du type susmentionné selon lequel un liquide à injecter est propulsé à haute vitesse par un générateur de pression de façon à percer la peau d'un patient humain ou d'un animal. De tels dispositifs sont par exemple décrits dans les brevets américains US 4,596,556, US 4,722,728, US 4,874,367, US 4,966,581 et US 5,501,666. Pour assurer la stérilité et pour éviter la contamination des médicaments à injecter, certains appareils conventionnels tels que décrits dans les brevets US 4,874,367 et US 4,966,581 comportent des cartouches à usage unique. Tous les dispositifs figurant dans ces brevets sont très complexes et comportent un grand nombre de pièces. Ces dispositifs sont encombrants, coûteux et sont tout de même limités dans leurs performances, notamment en ce qui concerne la pression d'injection et le diamètre du jet qui sont typiquement de l'ordre de 70 à 400 bars et 80 à 130 µm, respectivement. Une pression insuffisante et un grand diamètre de jet augmentent la douleur et comportent le risque, surtout chez les patients ayant une peau très résistante, qu'une partie seulement du médicament est injectée. L'efficacité de l'injection est très importante, notamment pour les patients, tels que des diabétiques, qui doivent s'administrer des injections tous les jours.

Au vu des inconvénients mentionnés ci-dessus, un but de la présente invention est de réaliser un dispositif d'injection hypodermique sans aiguille, stérile, efficace et fiable. En outre, il est avantageux de réaliser un dispositif d'injection hypodermique qui est compact et peu coûteux.

Des buts de l'invention sont réalisés par le dispositif d'injection hypodermique selon la revendication 1.

Dans la présente invention, un dispositif d'injection hypodermique à usage unique pour l'administration sous-cutanée, sans aiguille, d'un produit liquide à injecter tel qu'un médicament, un vaccin ou une autre composition pharmaceutique, comporte une ampoule contenant le liquide à injecter et une substance compressible liquide ou solide, une partie de buse ayant un orifice, et des moyens de rétention permettant de maintenir, avant l'utilisation, la substance compressible comprimée à une pression suffisante pour propulser le produit liquide à travers l'orifice de manière à créer un jet de liquide ayant une vitesse suffisante pour traverser la peau d'un patient.

La substance compressible peut par exemple être une substance viscoélastique, telle qu'une huile polysiloxane qui est peu coûteux et qui présente une grande capacité de compressibilité élastique. Le polysiloxane peut être comprimé jusqu'à 2000 bars avec une réduction de volume de 15%. En raison de la très haute pression et le faible diamètre de l'orifice, la vitesse du jet de liquide est supersonique.

Un élément de transmission de pression, tel qu'un piston, une membrane ou une paroi déformable, peut être disposé entre une partie de l'ampoule contenant le liquide à injecter et une partie contenant la substance compressible.

Si l'élément de transmission de pression est un piston, le moyen de rétention peut être conçu sous forme d'une tige de rétention du piston, cette tige s'étendant du piston jusqu'à une partie de fixation du récipient. Une partie d'ancrage de la tige peut être retenue à la partie de fixation de récipient par sertissage de la partie du récipient sur la tige, par soudure, par poinçonnage ou par d'autres moyens mécaniques. Un sertissage de la partie de fixation sur la partie d'ancrage de la tige est avantageux de par sa simplicité et la bonne étanchéité qu'elle confère à l'extrémité arrière du dispositif. La tige peut comporter une zone de rupture permettant de séparer la partie d'ancrage du reste pour libérer le piston.

La zone de rupture peut être une zone affaiblie et/ou rendue moins ductile, de sorte qu'en pliant la tige, elle se romp dans cette zone. On peut, par exemple, affaiblir cette zone de rupture par une rainure, des trous ou une entaille. La zone de rupture peut être rendue moins ductile par un procédé de trempe, notamment dans le cas d'une tige en alliage d'acier. La trempe peut être effectuée par un échauffement local par laser, ultrason, induction électromagnétique ou par d'autres moyens, suivi par un refroidissement.

Le moyen de rétention peut être conçu sous forme d'un bouchon bloquant l'orifice de la partie de buse, tel qu'un bouchon en cire qui peut être dégagé en appliquant un micro-échauffement local. Dans une telle forme d'exécution, le piston qui est flottant, ou la paroi déformable, se déplace au moment où l'orifice est dégagé du fait de la chute de pression dans la partie de l'ampoule contenant le liquide à injecter.

Dans une autre forme d'exécution, les parties de l'ampoule contenant le liquide à injecter et la substance compressible sont séparées par un passage de section réduite qui peut être bouché par divers moyens, soit par un moyen mécanique, soit par un bouchon de cire capable de fondre, ces éléments formant des moyens de rétention.

Dans ces formes d'exécution, le liquide à injecter est à pression atmosphérique jusqu'au moment où le passage entre les parties est libéré des moyens de rétention et l'élément de transmission de pression entre ces parties est propulsé par l'expansion de la substance compressible.

Dans une autre forme d'exécution, la partie contenant le liquide à injecter est entourée par une paroi déformable disposée à l'intérieur de la partie contenant la substance compressible et les moyens de rétention comportent un bouchon fermant l'orifice de la partie de buse. Au moment du dégagement des moyens de rétention, la paroi déformable de la partie contenant le liquide à injecter est écrasée sous la pression de la substance compressible.

Dans une autre forme d'exécution, la partie contenant la substance compressible se trouve à l'intérieur de la partie contenant le liquide à injecter et au moment du dégagement des moyens de rétention, la paroi déformable de la partie contenant la substance compressible subit une expansion à l'intérieur de la partie contenant le liquide à injecter de façon à le propulser hors de l'ampoule par l'orifice de la buse. La substance compressible subit donc une expansion de façon à occuper le volume de la partie contenant le liquide à injecter. La substance compressible peut occuper un volume continu ou plusieurs volumes séparés, par exemple sous forme de plusieurs capsules ou d'un grand nombre de microcapsules. Ces capsules peuvent par exemple comprendre une membrane entourant la substance compressible, telle qu'un liquide viscoélastique comme le polysiloxane, ou simplement consister en une matière solide, tel que du caoutchouc.

L'ampoule peut comporter un récipient en acier inox qui peut être muni, sur sa surface intérieure, d'une couche de métal précieux (par exemple l'or, le platine, le palladium) ou d'un polymer tel que du teflon. La couche intérieure permet de maintenir la pureté et la stérilité du médicament. D'autre part, la couche permet de faciliter le glissement du piston, le cas échéant, et d'améliorer l'étanchéité. L'étanchéité entre les parties contenant la substance compressible et le produit liquide peut aussi être améliorée en munissant l'intérieur de la partie de l'ampoule contenant la substance compressible d'une couche polymérique ou élastique, telle que du caoutchouc, entourant la substance compressible. Il est à noter que les huiles polysiloxanes sont très avantageuses par rapport à un gaz, d'une part puisque la viscosité, qui peut être très élevée dépendant du poids moléculaire de l'huile, réduit les exigences en ce qui concerne l'étanchéité et, d'autre part puisque une grande partie de l'énergie de compression peut être utilisée.

La partie de buse peut comporter une pièce séparée montée dans l'ampoule, ou peut faire partie intégrante avec la paroi formant l'ampoule, ou tout au moins la partie contenant le liquide à injecter.

L'orifice de la partie de buse peut avoir un diamètre de l'ordre de grandeur de 10 à 80 microns, au moins sur une longueur définie, de sorte que le jet de liquide reste cohérent sur quelques millimètres. Si le déplacement du piston entre le début et la fin de l'injection correspond à une variation de volume de la substance compressible de 7,5 %, ceci correspond à une variation de 1000 bars pour du polysiloxane. Si le polysiloxane est comprimé à 2000 bars à l'état initial, la fin du jet de liquide est propulsée par une pression de 1000 bars à une vitesse supersonique. Une si grande pression combinée avec un jet très fin permet d'injecter le liquide à travers la peau de façon extrêmement fiable et sans douleur. D'autre part, l'onde de choc supersonique cause la dégradation du jet en gouttelettes à quelques millimètres de la buse et accroît ainsi la sécurité d'utilisation.

La partie de l'ampoule contenant le liquide à injecter peut avoir un diamètre plus faible que la partie contenant la substance compressible, le piston comprenant une première partie et une deuxième partie ayant des diamètres adaptés aux diamètres de ces parties respectivement, de sorte qu'il y a une multiplication de pression égale essentiellement au rapport des sections transversales de ces parties.

La substance compressible peut être mise sous pression en l'injectant dans le récipient sous pression, ou en l'injectant à pression atmosphérique ou à basse pression dans l'ampoule et en déformant la partie de l'ampoule contenant la substance compressible de manière à réduire son volume de contenance.

D'autres buts et aspects avantageux de l'invention ressortiront de la description et des revendications ci-après et des dessins annexés, dans lesquels:
la Fig. 1 est une coupe longitudinale d'un dispositif d'injection en l'état initial selon une première forme d'exécution de l'invention;
la Fig. 2 est une coupe longitudinale de la première forme d'exécution pendant son utilisation;
la Fig. 3 est une vue montrant l'utilisation d'un dispositif d'injection selon l'invention par un patient;
la Fig. 4 est une coupe longitudinale d'une deuxième forme d'exécution;
la Fig. 5 est une coupe longitudinale partielle d'une partie de tige montrant la zone de rupture;
la Fig. 6 est une coupe selon les lignes VI-VI de la Fig. 5;
la Fig. 7 est une coupe longitudinale d'une partie d'une tige de rétention selon une autre forme d'exécution, montrant la zone de rupture;
la Fig. 8 est une coupe selon les lignes VIII-VIII de la Fig. 7;
la Fig. 9 est une coupe longitudinale d'une troisième forme d'exécution d'un dispositif selon l'invention;
la Fig. 10 est une vue d'une quatrième forme d'exécution;
la Fig. 11 est une coupe selon les lignes XI-XI de la Fig. 10;
la Fig. 12 est une vue d'une cinquième forme d'exécution;
la Fig. 13 est une vue en coupe partielle longitudinale de la cinquième forme d'exécution avec un bouton d'actionnement et un support pour positionner l'extrémité du dispositif contre la peau du patient;
la Fig. 14 est une coupe longitudinale partielle montrant un moyen de rétention du piston;
la Fig. 15 est une coupe longitudinale d'une partie du dispositif montrant une autre variante d'un moyen de rétention;
la Fig. 16 est une coupe longitudinale similaire à la Fig. 15 après actionnement du piston;
la Fig. 17 est une coupe longitudinale d'une sixième forme d'exécution d'un dispositif d'injection selon l'invention;
la Fig. 18 est une coupe longitudinale d'une septième forme d'exécution d'un dispositif d'injection selon l'invention;
la Fig. 19 est une coupe longitudinale d'une huitième forme d'exécution d'un dispositif d'injection selon l'invention;
la Fig. 20 est une coupe longitudinale d'une neuvième forme d'exécution d'un dispositif d'injection selon l'invention;
la Fig. 21 est une coupe longitudinale d'une dixième forme d'exécution d'un dispositif d'injection selon l'invention;
la Fig. 22 est une coupe longitudinale d'une onzième forme d'exécution d'un dispositif d'injection selon l'invention;
la Fig. 23 est une coupe longitudinale d'une douzième forme d'exécution d'un dispositif d'injection selon l'invention;
la Fig. 24 est une coupe longitudinale d'une treizième forme d'exécution d'un dispositif d'injection selon l'invention;
la Fig. 25 est une coupe longitudinale d'une quatorzième forme d'exécution d'un dispositif 'injection selon l'invention avant l'utilisation;
la Fig. 26 est une coupe longitudinale de la quatorzième forme d'exécution pendant l'utilisation;
la Fig. 27 est une coupe longitudinale d'une quinzième forme d'exécution d'un dispositif d'injection selon l'invention avant l'utilisation;
la Fig. 28 est une coupe longitudinale de la quinzième forme d'exécution pendant l'utilisation;
la Fig. 29 est une coupe longitudinale d'une seizième forme d'exécution d'un dispositif d'injection selon l'invention;
la Fig. 30 est une coupe longitudinale d'une dix-septième forme d'exécution d'un dispositif d'injection selon l'invention;
la Fig. 31 est une coupe longitudinale d'une dix-huitième forme d'exécution d'un dispositif d'injection selon l'invention;
la Fig. 32 est une coupe longitudinale d'une dix-neuvième forme d'exécution d'un dispositif d'injection selon l'invention avant l'utilisation;
la Fig. 33 est une coupe longitudinale de la dix-neuvième forme d'exécution d'un dispositif d'injection selon l'invention pendant l'utilisation;
la Fig. 34 est une coupe longitudinale de la vingtième forme d'exécution d'un dispositif d'injection selon l'invention pendant l'utilisation;
la Fig. 35 est une coupe longitudinale de la vingt-et-unième forme d'exécution d'un dispositif d'injection selon l'invention;
les Figures 36 et 37 sont des coupes longitudinales de la vingt-deuxième forme d'exécution d'un dispositif d'injection selon l'invention; et
la Fig. 38 est une coupe longitudinale de la vingt-troisième forme d'exécution d'un dispositif d'injection selon l'invention.

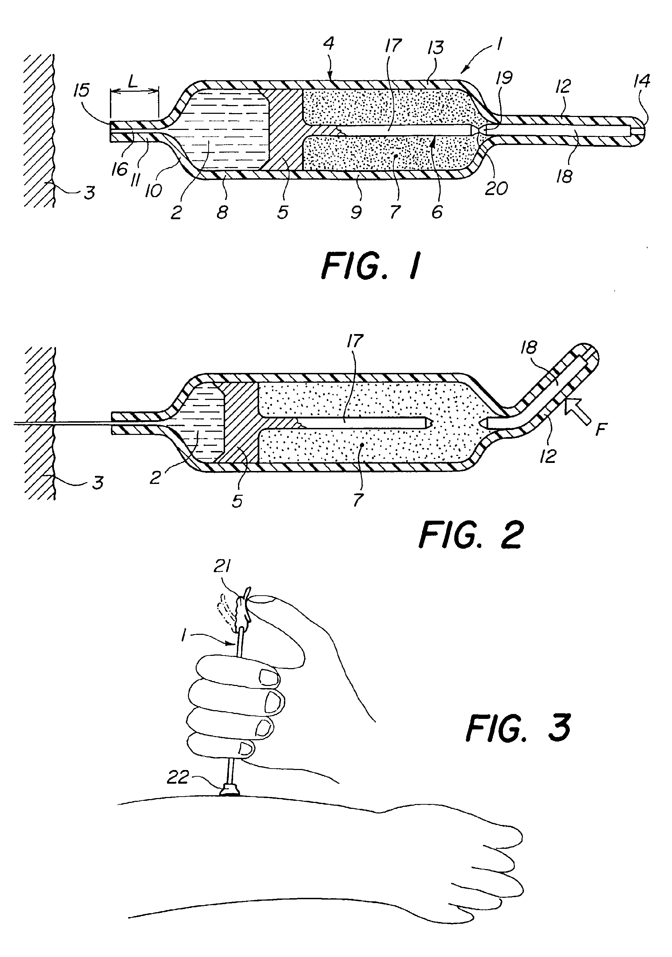

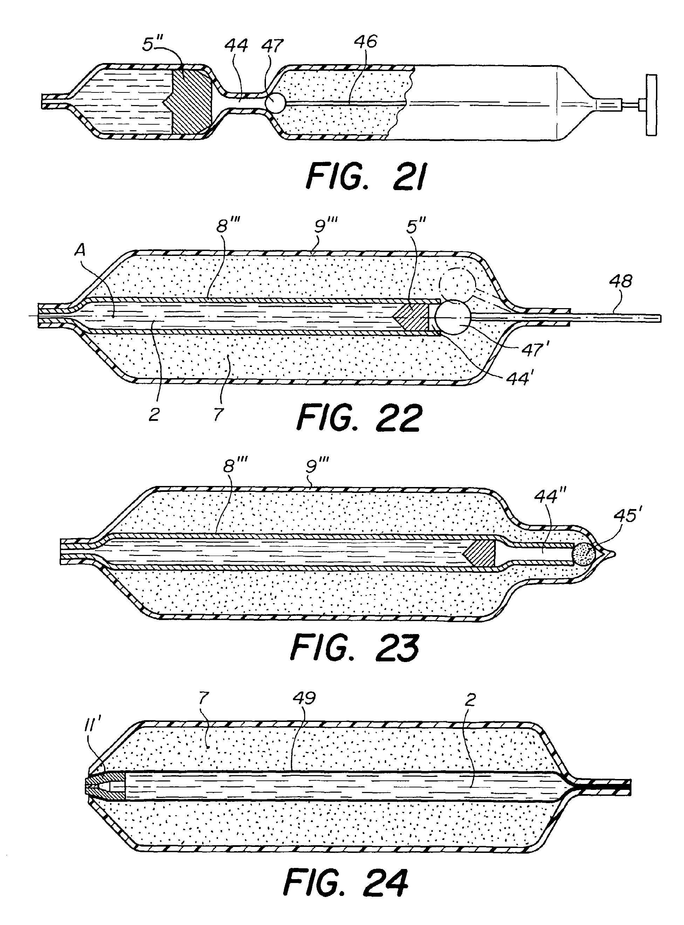

En se référant aux Figures 1 à 3, un dispositif d'injection 1 pour l'administration d'un liquide 2 sous la peau 3 d'un patient humain ou d'un animal comporte une ampoule 4, un élément de transmission de pression en forme d'un piston 5, un moyen de rétention 6 et une substance compressible 7. L'ampoule 4 comporte une partie 8 contenant le liquide à injecter et une partie 9 contenant la substance compressible. Le dispositif comporte en outre une partie de col 10 et une partie de buse 11 qui peuvent être formées d'une seule pièce avec la partie 8 contenant le liquide à injecter. La partie de buse peut aussi comporter une pièce séparée logée dans l'ampoule telle que montrée dans la Fig. 24 sous la référence II'. L'ampoule d'injection peut aussi comporter une partie de fixation 12 faisant partie intégrale avec la paroi 13 de l'ampoule. La paroi 13 du récipient s'étend ainsi intégralement d'une extrémité arrière 14 à une extrémité avant 15.

La partie de buse 11 a un orifice 16 qui peut avoir un diamètre de l'ordre de grandeur entre 5 et 100 microns, mais qui est de préférence aux environs de 20 à 50 microns. L'orifice s'étend sur une longueur L qui est de préférence entre environ deux et cinq fois le diamètre de l'orifice. Le rapport entre la longueur L et le diamètre de l'orifice permet de produire un jet de liquide cohérent sur une distance permettant l'injection hypodermique fiable, mais qui se déstabilise après quelques millimètres de façon à rendre le jet inoffensif. En d'autres mots, le rapport entre la longueur et le diamètre de l'orifice permet de régler la cohérence du jet, afin qu'il soit suffisamment cohérent pour permettre une injection sous-cutanée efficace et fiable sans être trop cohérent pour des raisons de sécurité.

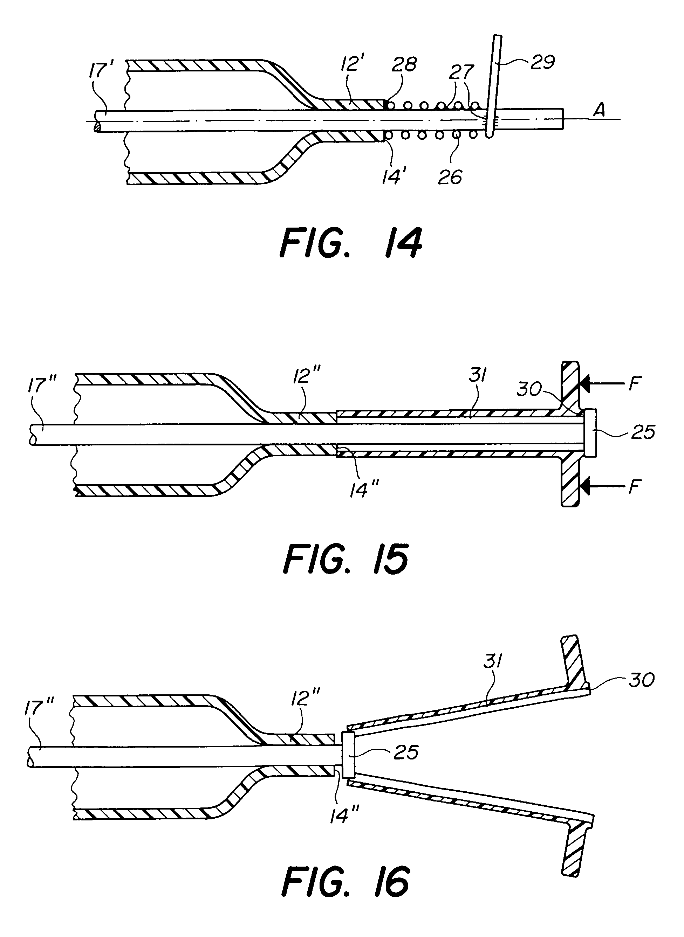

Les moyens de rétention 6 comportent, dans cette forme d'exécution, une tige de rétention 17 attachée au, ou solidaire du piston 5 et s'étendant jusqu'à une partie d'ancrage 18 fixée à la partie de fixation 12 de l'ampoule 4. La partie d'ancrage de la tige peut être fixée à la partie de fixation par sertissage ou par d'autres moyens tels que poinçonnage, soudage ou par la réalisation d'un rebord 25, tel que montré dans la Fig. 15.

Entre le piston 5 et la partie d'ancrage 18, la tige est munie d'une zone de rupture 19 permettant la libération du piston 5 par rupture de la tige en cette zone. La zone de rupture comporte une rainure 20 pour l'affaiblissement de la section de tige. La zone de rupture peut aussi être fragilisée par une trempe localisée. La trempe peut être effectuée par un échauffement local, par laser, ultrasons ou par induction électromagnétique, suivi d'un refroidissement rapide. A cet effet, la tige de rétention 17 est de préférence en acier. Il est aussi possible de former le piston et la tige en silice (verre) ou en d'autres matériaux, tels que l'epoxyde renforcé par des fibres de carbone ayant une fragilité suffisante pour pouvoir être rompu quand la tige est sollicitée mécaniquement (torsion ou flexion) dans un mouvement d'actionnement par l'utilisateur. Dans cette forme d'exécution, la zone de rupture 20 est proche de la partie de fixation de l'ampoule tel que illustré dans les Figures 2 et 3, de sorte qu'un fléchissement plastique de la partie de fixation romp la tige dans la zone de rupture.

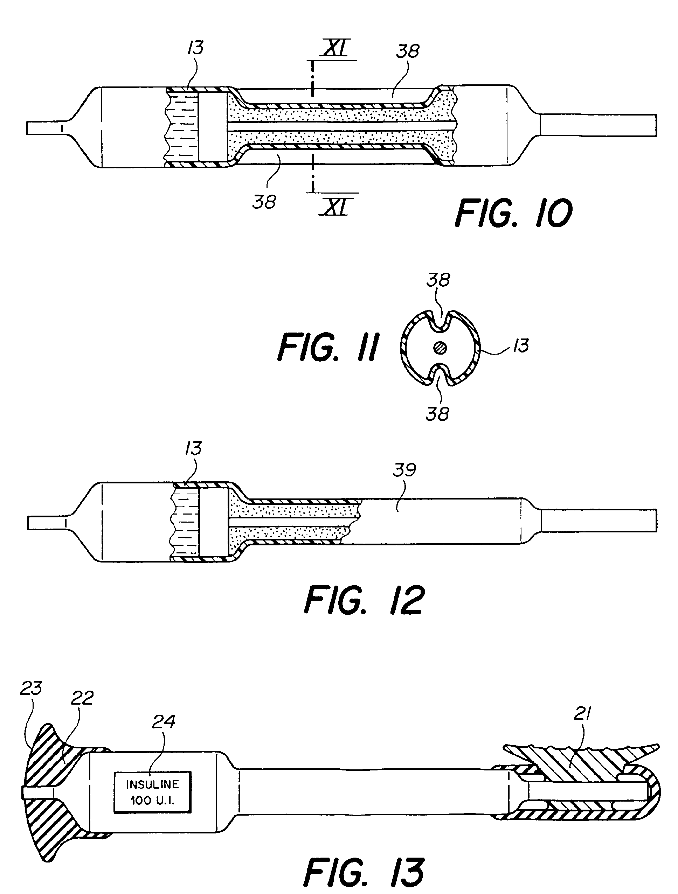

Pour faciliter ce fléchissement, le dispositif d'injection peut être muni d'un organe de poussée 21, tel que montré dans les Figures 3 et 13, enfilé sur la partie de fixation 12 du récipient à l'extrémité arrière.

La déformation plastique (permanente) de la partie de fixation 12 a l'avantage de fournir une indication claire à l'utilisateur que le dispositif a été utilisé. Pour le confort de l'utilisateur et un bon positionnement de l'extrémité 15 de la partie de buse 11, le dispositif d'injection comporte un support 22 tel que montré dans les Figures 3 et 13, par exemple en matière plastique, monté sur l'extrémité avant du récipient et disposant d'une surface d'appui 23.

Pour l'identification du produit, le dispositif peut en outre comporter une plaque signalétique 24, comme montrée dans la Fig. 13, indiquant le produit et sa composition ou sa quantité, etc.

La substance compressible peut avantageusement comprendre une huile polysiloxane. Les huiles polysiloxanes sont des liquides viscoélastiques limpides, clairs, inodores, insipides, résistants aux basses et hautes températures et peu coûteuses. Elles ne sont ni toxiques, ni dangereuses du point de vue physiologique et peuvent être utilisées en dermatologie et en cosmétique. Les huiles polysiloxanes présentent une faible variation de viscosité en fonction de la pression, ce qui est avantageux pour faciliter les échanges de fluides, mais elles ont une tensioactivité élevée de façon à être non-miscibles avec une solution d'eau. Elles présentent également une propriété lubrifiante entre les métaux et les polymères et le caoutchouc, ce qui est avantageux pour faciliter le glissement des éléments mobiles.

La famille des huiles polysiloxanes comporte entre autres les substances suivantes:
- polyméthylhydrogènesiloxane
- polydiméthylsiloxane
- polytriméthylsiloxane
- hexaméthylcyclotrisiloxane
- decaméthyltetrasiloxane
- hexaméthyldisiloxane (H 7310 - Witheco)
- octaméthyltrisiloxane (O 9816 - Witheco).

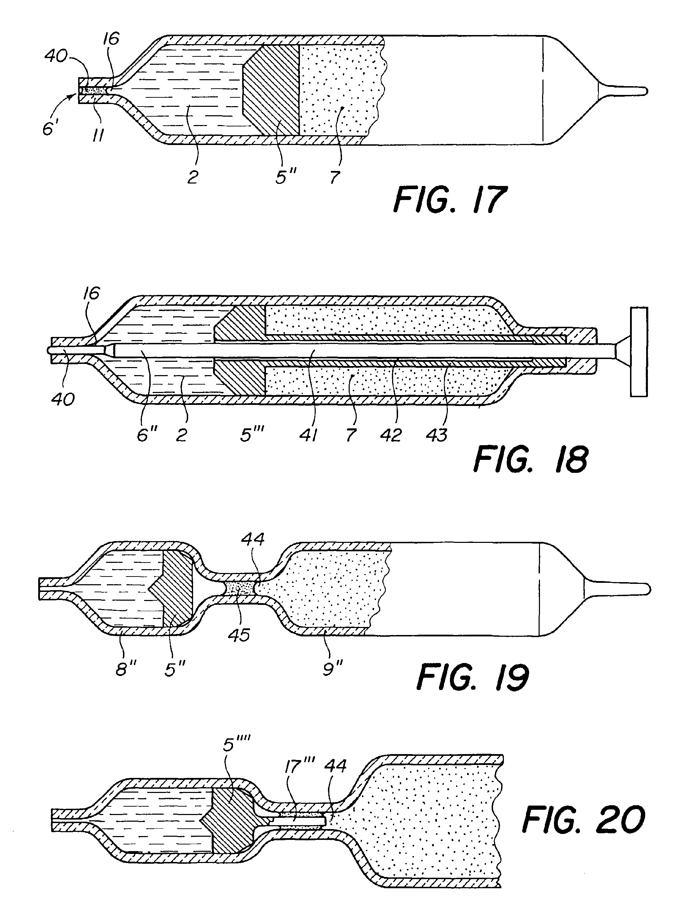

Une propriété intéressante de ces huiles polysiloxanes est que la viscosité diminue avec la vitesse de cisaillement de façon à permettre un écoulement rapide de ces huiles à travers de petits orifices. Les huiles polysiloxanes peuvent avoir des viscosités allant de 0,6 jusqu'à 10⁷ centistokes selon le poids moléculaire. Cette propriété permet entre autres de choisir l'huile selon la forme d'exécution, à savoir les formes d'exécution qui nécessitent un écoulement à travers des passages de petites sections, telles que celles montrées dans les figures 19 à 23, 26 et 37, peuvent comporter une huile polysiloxane à faible viscosité. Les autres formes d'exécution, notamment celles munies d'un piston, peuvent comporter des huiles polysiloxanes à grand viscosité, réduisent ainsi les exigences concernant l'étanchéité.

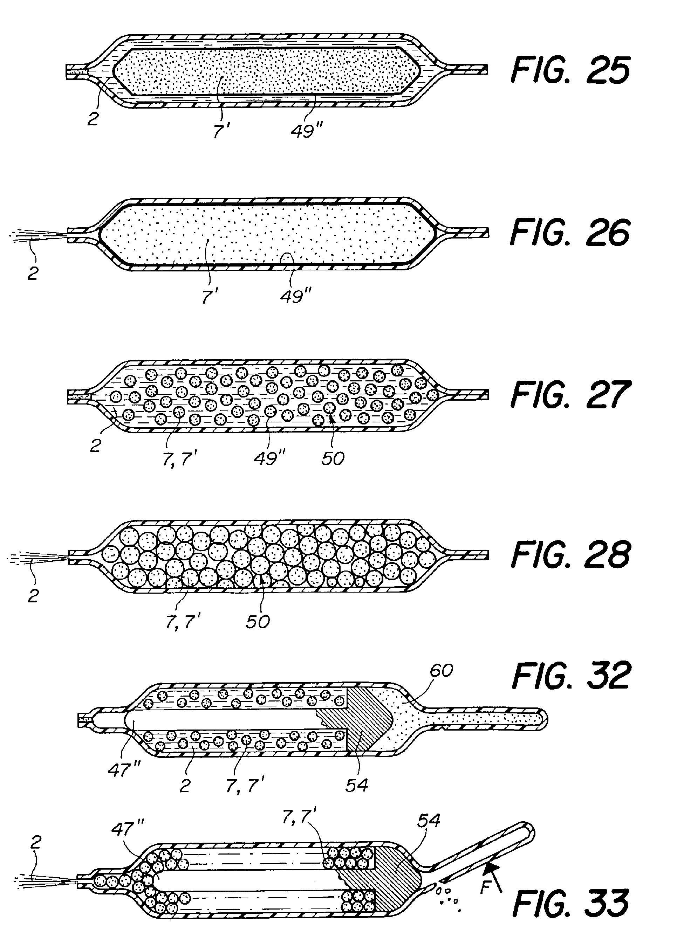

La substance compressible peut aussi comprendre un solide élastique, tel qu'un caoutchouc silicone vulcanisable, par exemple de type SilGel® 6/2 de Wacker-Chemie, présentant de très bonnes propriétés de compressibilité. L'utilisation d'une substance compressible solide est avantageux pour les formes d'exécution des figures 25 à 28 qui seront décrites plus loin.

Les huiles polysiloxanes peuvent être comprimées élastiquement sous une pression d'environ 2000 bars avec une réduction de volume d'environ 15 %. A titre d'exemple, si le volume du liquide à injecter est de 0,1 ml, et la pression minimale à la fin d'injection est de 1000 bar, le volume non-comprimé de polysiloxane est de 1,3 ml. Le dispositif selon l'invention est non seulement extrêmement compact, mais permet aussi d'injecter du liquide à des pressions entre 2000 et 1000 bars, ce qui permet la production d'un jet très fin à vitesse supersonique et une injection hypodermique très fiable.

En outre, le dispositif d'injection selon l'invention comporte très peu de pièces, ce qui permet la production du dispositif à faible coût et une bonne adaptation à l'usage unique garantissant ainsi la stérilité, la facilité de stockage et de distribution et la simplicité et fiabilité d'utilisation.

La Fig. 14 montre une autre variante d'un moyen de rétention, selon laquelle la tige de rétention 17' est fixée à la partie de fixation 12' par un fil hélicoïdal 26 qui est soudé à la tige en des points de soudure 27, 28. La tige 17' ne comporte pas de zone de rupture et est montée de façon coulissante dans la partie de fixation 12'. L'actionnement du dispositif s'effectue en applicant une force de rotation sur l'extrémité d'actionnement 29 du ressort autour de l'axe longitudinal A pour rompre les points de micro-soudure 27, 28 et libérer la tige 17'.

La Fig. 15 montre une autre variante d'un moyen de rétention, selon laquelle la tige de rétention 17" qui est montée de façon coulissante dans la partie de fixation 12" du récipient, est retenue par engagement du rebord 25 contre l'extrémité 30 d'un tube fendu 31 qui s'appuie à l'autre extrémité sur l'extrémité arrière 14" du récipient. Une force axiale F appliquée sur les extensions latérales 32 permet de libérer le rebord 25 de l'extrémité 30 par une rotation des parties du tube 31, tel que montré dans la Fig. 16.

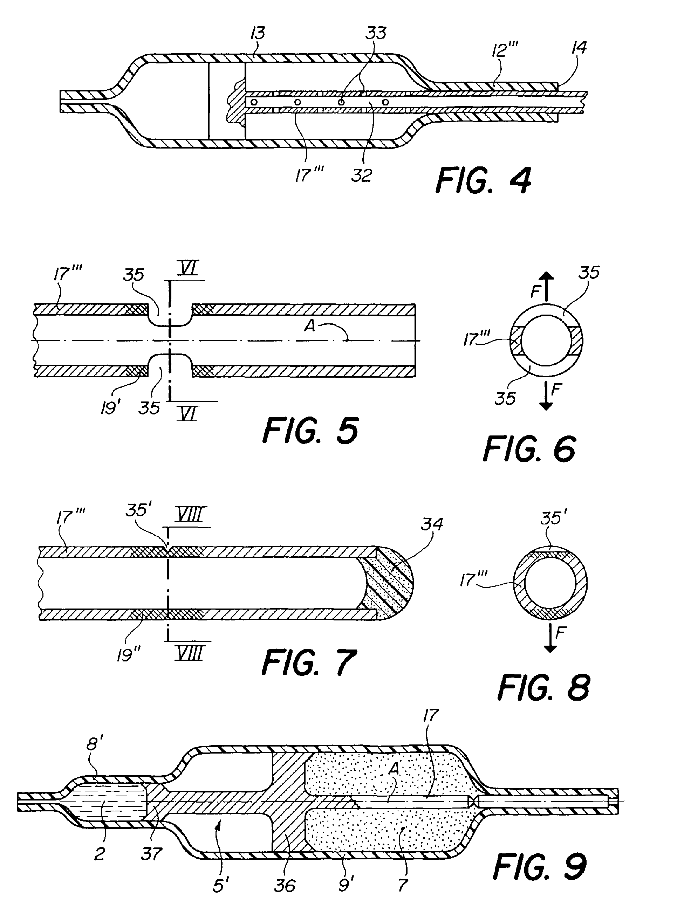

La Fig. 4 montre encore une autre forme d'exécution selon laquelle la tige de rétention 17"' comporte un passage central 32 et des orifices latéraux 33 pour permettre le remplissage de la partie de l'ampoule contenant la substance compressible depuis l'extrémité arrière 14 du dispositif. Après l'opération de remplissage, l'extrémité arrière du passage 32 peut être fermée par une goutte de soudure ou de colle 34 telle qu'illustrée à la Fig. 7, ou le tube 17"' peut être écrasé par une opération de sertissage ou d'emboutissage sur la partie de fixation 12"'.

Une tige en forme de tube 17"' peut avoir une zone de rupture 19', 19" affaiblie par des trous latéraux 35 tels que montrés dans les Figures 5 et 6, dans une zone trempée, donc fragilisée, ou par d'autres moyens d'affaiblissement tels qu'une entaille 35' montrée dans les Figures 7 et 8. Dans la variante montrée dans les Figures 5 et 6, la tige est rompue en appliquant une force transversale à l'axe longitudinale A sur l'un ou l'autre des côtés démunis d'un orifice, alors que dans la variante des Figures 7 et 8, la rupture ne s'effectue qu'en applicant une force de poussée sur le côté de la tige munie de l'entaille 35'.

Dans la Fig. 9, une troisième forme d'exécution de l'invention comporte un système multiplicateur de pression. Le système multiplicateur de pression est réalisé par une surface (en section transversale) d'une première partie 36 du piston 5' en contact avec la substance compressible 7 supérieure à la surface (en section transversale) d'une deuxième partie 37 du piston en contact avec le liquide à injecter 2. La partie de l'ampoule contenant la substance compressible 9' a donc un diamètre supérieur à la partie contenant le liquide à injecter 8'. La multiplication de pression est égale au rapport des surfaces de la section orthogonale à l'axe longitudinale A des parties de piston 36 et 37. Le multiplicateur de pression permet de raccourcir le dispositif, ou d'augmenter la pression d'injection, ou de diminuer la compression de la substance compressible, et permet ainsi un plus grand champ d'utilisation du dispositif.

Dans les Figures 10 à 12, la partie de l'ampoule contenant la substance compressible comporte des enfoncements 38 ou un diamètre réduit 39 réalisés après le remplissage de cette partie par la substance compressible. Une réduction de volume de cette partie par déformation de la paroi 13 peut résulter en toutes sortes de formes, l'essentiel étant de réduire le volume pour mettre sous pression la substance compressible. Une mise sous pression de ce type peut aussi être appliquée aux autres formes d'exécution. Ceci permet de remplir la partie de l'ampoule contenant la substance compressible à basse pression, facilitant ainsi les opérations de remplissage et de production du dispositif. Comme déjà mentionné, si la substance compressible est du polysiloxane, le volume peut être réduit de 15% tout en restant dans le domaine de compression essentiellement élastique, résultant en une pression de 2000 bars.

Dans la Fig. 17, une autre forme d'exécution est montrée selon laquelle les moyens de rétention 6' comportent un bouchon 40 fermant l'orifice 16 de la partie de buse 11, de sorte que la pression du liquide à injecter 2 est égale à la pression de la substance compressible 7 et que le piston 5" séparant le liquide et la substance est monté de façon flottante.

Le bouchon peut, par exemple, être en un matériau qui se décompose sous l'effet d'une sollicitation externe, tel qu'un matériau fondant, au moment de l'utilisation, est dégagée par micro-échauffement local de la partie de buse 11. La cire d'abeille ou la paraffine est un exemple d'un matériau fondant qui pourrait être utilisé dans la présente invention. L'orifice 16 ayant un diamètre très faible d'environ 50 µm ou moins, de la cire d'abeille ou de la paraffine suffit pour boucher cet orifice et pour résister à la pression de 4000 bar.

La Fig. 18 montre une autre forme d'exécution selon laquelle le piston 5"' est monté plus ou moins de façon flottante, de sorte que le liquide à injecter 2 est à la même pression que la substance compressible 7. Les moyens de rétention 6" comportent un bouchon 40' sur une tige 41 s'étendant jusqu'à l'extrémité arrière du récipient. Quand la tige est retirée vers l'arrière, le bouchon 40' dégage l'orifice 16 et le piston 5"' est propulsé par le liquide compressible 7. Le piston 5"' est muni d'un passage 42 pour la tige 41. Ce passage 42 peut être étanche, mais permet le coulissement du piston sur la tige. Le piston 5"' peut aussi être attaché à un tube 43 qui s'étend jusqu'à l'arrière du récipient pour améliorer l'étanchéité.

La Fig. 19 montre une autre forme d'exécution selon laquelle le piston 5" est flottant comme dans la forme d'exécution de la Fig. 17, mais la partie 8" de l'ampoule contenant le liquide à injecter et la partie 9" contenant la substance compressible communiquent à travers un passage de section réduit 44 qui est bloqué par un moyen de rétention 45 avant l'utilisation. Ce moyen de rétention peut être un bouchon en un matériau fondant, tel que de la cire, et qui est dégagé par micro-échauffement, tel que montré dans la Fig. 19. Le moyen de rétention peut aussi comporter une tige 17" qui est insérée dans le passage 44 et qui s'étend du piston 5"" tel que montré dans la Fig. 20. La tige 17"" est retenue par une substance fondante, par ex. de la paraffine qui peut être fondue par micro-échauffement. Il est aussi concevable de fixer la tige par un sertissage ou par un autre moyen mécanique, et de casser la tige pendant l'actionnement du dispositif en fléchissant la partie de l'ampoule contenant la substance liquide compressible par rapport à la partie de l'ampoule contenant le liquide à injecter.

Le bouchon peut aussi être constitué par une tige 46 munie d'un bouchon 47 à son extrémité bloquant le passage 44 tel que montré dans la Fig. 21. La tige est retirée pour dégager le passage 44 et pour actionner l'injection.

Au lieu d'avoir des parties de l'ampoule juxtaposées, il est aussi possible de positionner la partie de l'ampoule contenant le liquide à injecter à l'intérieur ou à l'extérieur de la partie de l'ampoule contenant la substance compressible. Du fait que la substance compressible occupe un volume plus grand que le liquide à injecter dans la plupart des applications, la partie de l'embout contenant le liquide à injecter est de préférence disposée à l'intérieur de la partie contenant la substance compressible, tel que montré dans les Figures 22 et 23, la partie contenant le liquide à injecter étant désigné par le numéro de référence 8"' et la partie contenant la substance compressible par le numéro de référence 9"'. Dans ces deux formes d'exécution, un piston est monté coulissant dans la partie contenant le liquide à injecter qui communique avec la partie contenant la substance compressible par un passage 44', 44" qui est bouché par un moyen de rétention pouvant être formé de matériaux fondants tels que de la cire d'abeille, tel que montré dans la Fig. 23, ou être constitué par un bouchon mécanique pouvant être dégagé par une rotation de la tige 48 autour d'un axe perpendiculaire au plan contenant l'axe longitudinale A ou, comme pour la variante montrée dans la Fig. 21, par un retrait de la tige 48 dans la direction longitudinale.

Dans la Fig. 24, l'élément de transmission de pression est constitué par une paroi déformable 49 qui sépare le liquide à injecter de la substance compressible 7, ceux-ci étant essentiellement à la même pression. Dans cette forme d'exécution, le moyen de rétention constitue un bouchon dans l'orifice de la buse qui peut, par exemple, être similaire au bouchon décrit par rapport à la forme d'exécution de la Fig. 17. En dégageant l'orifice pendant l'actionnement du dispositif, la paroi déformable 49 s'écrase sous la pression de la substance compressible. La paroi 48 peut être un tube métallique très mince ou être en un matériau plastique ou en caoutchouc.

Dans la Fig. 25, la partie contenant la substance compressible 7' est à l'intérieur de la partie contenant le liquide à injecter 2, de sorte qu'au moment de l'ouverture de l'orifice de la partie de buse, la substance compressible se détend élastiquement pour remplir le volume de la partie contenant le liquide à injecter et donc de l'expulser par l'orifice de la buse tel que montré dans la Fig. 26.

La substance compressible 7' peut être une huile polysiloxane enfermée dans une membrane 49" élastiquement ou plastiquement déformable, ou peut-être un matériau solide, tel que du caoutchouc silicone vulcanisable. Dans une telle forme d'exécution, l'élément de transmission de pression peut être considéré comme la surface ou la paroi extérieure du bloc de caoutchouc.

Les figures 27 et 28 montrent une forme d'exécution similaire à celle des figures 25 et 26 respectivement, sauf que la substance compressible ne forme pas un volume continu mais est divisé en plusieurs capsules enfermant du polysiloxane ou plusieurs boules de caoutchouc ou d'autres substances compressibles, solides ou liquides, dans le produit liquide à injecter avant l'utilisation, tel que montré dans la Fig. 27. En ouvrant l'orifice de la buse, les microcapsules ou boules se détendent et expulsent le produit liquide à injecter, tel que montré dans la Fig. 28. Dans cette forme d'exécution, l'élément de transmission de pression peut être considéré comme une multitude de parois ou de surfaces extérieures des capsules ou des boules 50, respectivement.

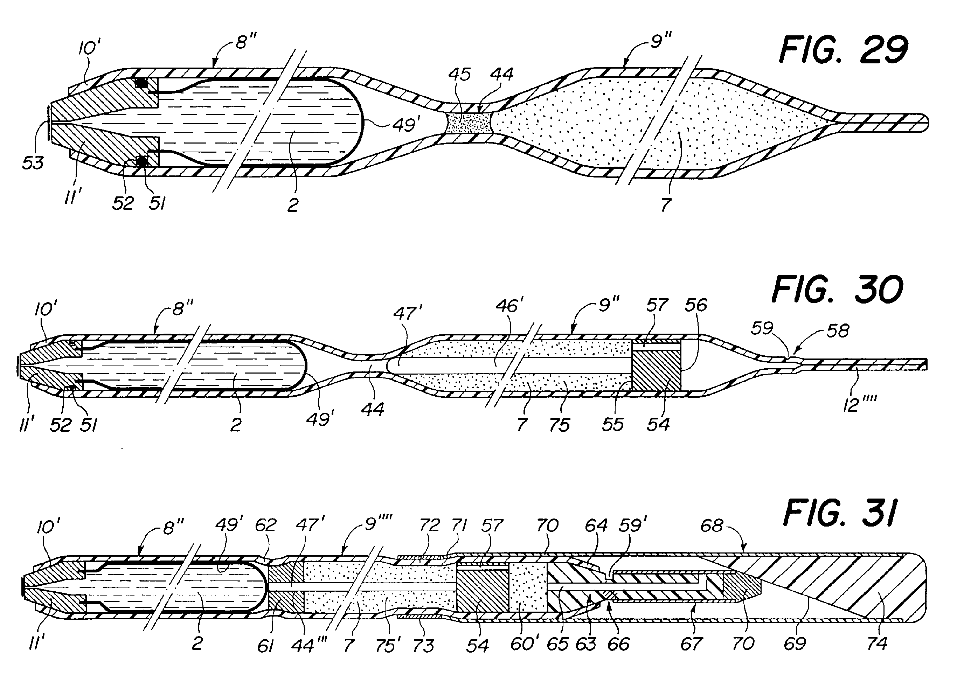

La Fig. 29 montre une forme d'exécution similaire à celle de la Fig. 19 en ce qu'elle comporte une partie d'ampoule 8" contenant le liquide à injecter et une partie d'ampoule 9" contenant la substance compressible, communiquant à travers un passage de section réduit 44 bloqué par un moyen de rétention 45. Dans cet exemple, le moyen de rétention est constitué par un bouchon en un matériau fondant, tel que la cire, et qui peut être dégagé par un échauffement local. Cette forme d'exécution diffère de celle de la Fig. 19 en ce qu'elle ne comporte pas de piston, le liquide à injecter 2 étant entouré par une membrane déformable 49', par exemple en polyéthylène. La membrane 49' peut former la paroi d'une cartouche stérile comprenant aussi la partie de buse 11' et contenant le liquide à injecter. La cartouche est assemblée dans la paroi de l'ampoule et fixée, par exemple, par sertissage de la partie de col 10'. Pour assurer l'étanchéité entre la partie de buse et la surface intérieure de la partie d'ampoule contenant le liquide à injecter 8", on peut inclure un joint torique en caoutchouc monté dans une rainure 52 sur le pourtour d'une partie arrière de la partie de buse. Une feuille protectrice 53, par exemple un film polyéthlène d'environ 10 µm d'épaisseur, peut être collée sur l'extrémité avant de la partie de buse pour assurer la stérilité et l'étanchéité de la cartouche. Avantageusement, la cartouche est remplissable par des produits liquides dans des conditions adaptées aux grands volumes et aux conditions de stérilité et dans des dosages adaptés à l'utilisation voulue, sans influer sur la construction des autres parties de l'ampoule. La cartouche peut ensuite être assemblée dans la partie de l'ampoule 8" contenant le liquide à injecter.

La Fig. 30 montre une autre forme d'exécution comportant une partie de l'ampoule contenant le liquide à injecter et une partie de buse identique à celles de la Fig. 29, mais des moyens de rétention de la substance compressible 7 différents. Les moyens de rétention comportent un bouchon 47' sur une tige 46' attachée à un piston 54 dans la partie 9" de l'ampoule contenant la substance compressible 7. Avant l'utilisation, le bouchon 47' bloque le passage 44 reliant les parties 8", 9" de l'ampoule. La substance compressible 7, qui est de préférence une substance liquide telle qu'une huile polysiloxane décrite ci-dessus, est maintenue sous pression par la fermeture du passage 44 par le bouchon 47', la pression des deux côtés avant et arrière 55, 56 du piston 54 étant égalisé par un ou plusieurs orifices ou passages 57 traversant le piston 54. Le côté avant de la tige 46' a une surface plus faible que le côté arrière 56, de sorte que le piston 54, la tige 46' et le bouchon 47 sont soumis à une force résultant vers l'avant (i.e. dans la direction du passage 44) de façon à assurer que le passage 44 soit bouché par le bouchon 47'. Le volume de la substance compressible 7 dans la partie avant entre le piston et le bouchon 47' a un volume pour expulser le volume spécifié de liquide à injecter par décompression, tandis que la partie arrière de l'ampoule entre le côté arrière 56 du piston et l'extrémité arrière 12"" a un volume très faible, mais permet un déplacement du piston 54 vers l'arrière suffisamment pour dégager le bouchon 47' du passage 44. L'ampoule est munie d'une zone 58 qui peut être fragilisée par une trempe suivie d'un refroidissement, et/ou en taillant une rainure ou entaille 59 dans la paroi de l'ampoule en cette zone. L'utilisateur appuie sur la partie arrière 12"" pour la fléchir et entraîner la rupture de l'ampoule dans la zone 58 créant ainsi un passage pour la décompression de la substance compressible dans la partie arrière 60 de l'ampoule. La chute de pression dans la partie arrière 60 entraîne le déplacement du piston vers l'arrière, et donc le dégagement du passage 44. L'orifice 57 traversant le piston 54 étant très petit et la viscosité de la substance compressible étant relativement faible, le liquide à injecter 2 est propulsé hors de l'ampoule par la détente de la substance compressible dans la partie avant, avant que la chute de pression résultant de la communication de la substance compressible avec la partie arrière 60 par le passage 57 ne soit trop importante.

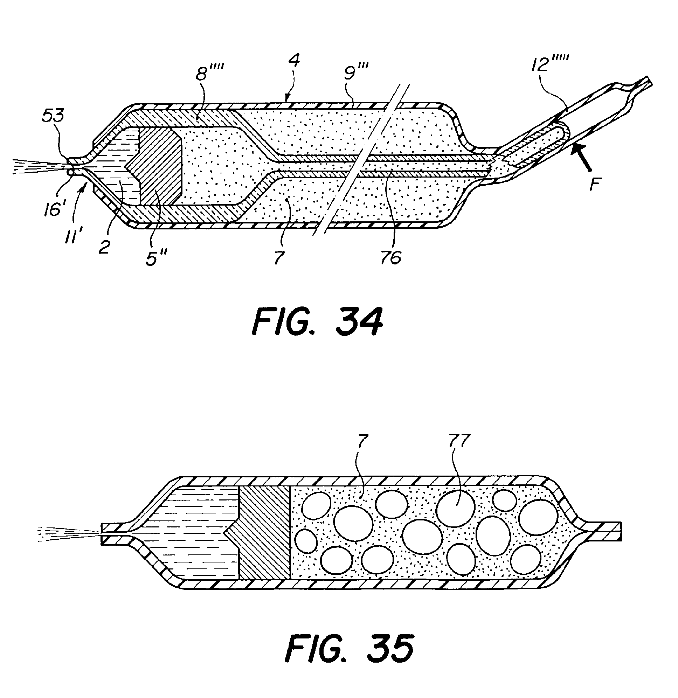

La Fig. 31 montre une autre forme d'exécution similaire à la forme d'exécution de la Fig. 30. La partie de l'ampoule contenant le liquide à injecter ainsi que la partie de buse sont essentiellement similaires ou identiques aux éléments correspondants des formes d'exécution des figures 29 et 30. Le piston 54 ainsi que la tige 46' et le bouchon 47' peuvent aussi être essentiellement similaires ou identiques à la variante de la Fig. 30. La forme d'exécution de la Fig. 31 diffère de la forme d'exécution de la Fig. 30 principalement dans la partie de passage 44' et dans la partie arrière 60' de l'ampoule. Le passage 44' est formé dans un insert 61, qui peut par exemple être une pièce en plastique, fixée dans l'ampoule, par exemple par retrécissement ou par sertissage 62, de la paroi de l'ampoule sur l'insert 61 à l'emplacement de l'insert. Cette construction permet d'avoir un bouchon 47' s'étendant sur une certaine longueur dans le passage 44', de sorte que l'actionnement du dispositif requiert un déplacement du piston 54 de quelques millimètres augmentant ainsi la fiabilité et la sécurité par rapport à la forme d'exécution de la Fig. 30. Cette forme d'exécution permet ainsi d'assurer une bonne étanchéité entre les parties de l'ampoule contenant le liquide à injecter 2 et la substance compressible 7. La partie arrière 60' de l'ampoule est fermée par un bouchon arrière 63 qui peut être fixé à l'intérieur de la partie 60' par un col arrière 64. Le bouchon arrière 63 est démuni d'un orifice ou passage 65 reliant la partie arrière 60' à une zone de rupture 66 comportant une entaille 59'. En pliant une partie d'extrémité arrière 67 du bouchon 63, il se casse dans la zone de rupture 66 de sorte que le passage 65 communique avec l'extérieur. La substance compressible contenue dans la partie arrièe 60' de l'ampoule est expulsée par le passage 65, de sorte que le piston 54 est déplacé vers l'arrière et dégage le bouchon 47' du passage 44' entre les parties de l'ampoule 8", 9"". La chute de pression dans la partie 75' contenant la substance compressible 9"" due au passage 57 dans le piston 54 est minimisée du fait que lorsque le piston 54 bute contre le bouchon arrière 63 pendant l'actionnement, le passage d'écoulement vers l'arrière devient très restreint. Il est à noter que l'insert 61 peut être conçue de façon à se déplacer comme un piston vers la partie contenant le liquide à injecter quand le bouchon 47' est retiré du passage 44'. A cet effet, la libération du passage 44' permet l'écrasement radial de l'insert, de sorte qu'il puisse passer à travers la section réduite par le rétrécissement 62.

Le dispositif de la Fig. 31 comporte en outre un élément d'actionnement 68 sous la forme d'un poussoir comportant une surface oblique 69 pouvant être engagée contre une surface complémentaire 70 de la partie d'extrémité arrière 67 du bouchon arrière 63 pour le fléchir et causer sa rupture. L'élément d'actionnement 68 comporte une partie de tube 70 qui est montée de façon coulissante sur l'extérieur de la partie arrière de l'ampoule. Une extrémité avant 71 de cet élément 68 est inclinée légèrement vers l'intérieur pour s'engager dans un rétrécissement léger 72 de l'ampoule autour duquel est positionnée une bague déformable 73. Le rétrécissement et la bague permettent de positionner et retenir l'élément d'actionnement 68 avant l'utilisation. Quand l'utilisateur pousse sur le bouton 74, la bague 73 est déplacée vers l'avant en s'étendant élastiquement ou plastiquement pour sortir du rétrécissement 72, permettant ainsi le coulissement de l'élément d'actionnement. Le déplacement de la bague ainsi que l'élément d'actionnement permettent aussi d'indiquer que l'ampoule a été utilisée.

Les figures 32 et 33 montrent une autre forme d'exécution comprenant un piston 54 avec un orifice 57 s'étendant entre une partie arrière 60 de l'ampoule et une partie contenant le liquide à injecter et la substance compressible. Le piston ainsi que la partie arrière du dispositif de cette forme d'exécution peuvent être similaires ou identiques à la forme d'exécution montrée dans la Fig. 30. Cette forme d'exécution diffère des formes d'exécution selon les figures 30 et 31 en ce que les parties de l'ampoule contenant le liquide à injecter et la substance compressible sont confondues de façon similaire aux formes d'exécution des figures 25 à 28. La rupture de la partie arrière de l'ampoule telle que montrée à la Fig. 33 cause le déplacement du piston 54 vers l'arrière entraînant la libération du passage dans la partie de buse par le déplacement du bouchon 47". La substance compressible 7, 7' peut être dans la même forme que celles prévues pour les formes d'exécution des figures 27 et 28, le liquide à injecter 2 remplissant le volume restant.

La Fig. 34 montre une autre forme d'exécution selon laquelle la partie 8"" contenant le liquide à injecter 2 est montée à l'intérieur de la partie 9"' contenant la substance compressible 7, de façon similaire aux formes d'exécution des figures 21 et 22, sauf que la partie contenant le liquide à injecter est en un matériau peu ductile, tel que du verre, et qui peut être brisé pour permettre l'introduction de la substance compressible dans la partie contenant le liquide à injecter derrière le piston 5". Dans cette forme d'exécution, un tube 76 faisant partie intégrante de la paroi de la partie 8"" fait office de bouchon et de passage à section réduite à titre de comparaison avec les formes d'exécution des figures 21 et 22. Le tube 76 est brisé dans sa partie arrière par le fléchissement de l'extrémité arrière 12""' du dispositif. D'autres moyens pour briser le tube peuvent néanmoins être réalisés. Par exemple, la partie contenant le liquide à injecter peut être munie d'un élément en un matériau magnétique, tel qu'une bague à l'extérieur ou une tige à l'intérieur du tube. La force magnétique résultant d'un aimant amené à proximité de l'élément magnétique permet de fléchir et de briser le tube.

Dans la forme d'exécution de la Fig. 34, le liquide à injecter peut être chargé dans la partie contenant le liquide à injecter de façon stérile et à un endroit séparé de l'assemblage de l'ampoule, de façon similaire aux formes d'exécution des figures 29 à 31. Les avantages sont les mêmes, c'est-à-dire la cartouche est remplissable par des liquides dans des conditions adaptées aux grands volumes et aux conditions de stérilité et dans des dosages adaptés à l'utilisation voulue, sans influence sur la construction des autres parties de l'ampoule. La partie 8"" de l'ampoule contenant le liquide à injecter peut ensuite être assemblée dans la partie 9"' de l'ampoule contenant la substance compressible. Dans cette forme d'exécution, la partie de buse 11' munie de l'orifice 16' est formée intégralement avec la paroi de la partie 8"" contenant le liquide à injecter.

Dans la forme d'exécution de la Fig. 34, le pic de pression (coup de bélier) est obtenu par l'accélération du liquide compressible dans le tube 76.

Dans la forme d'exécution de la Fig. 35, le pic de pression (coup de bélier) est obtenu par la détente rapide du liquide compressible 7, suivie par la détente du gaz liquéfié dissous s'opérant par un changement de phase.

La substance compressible liquide peut être une huile polysiloxane mise sous une pression moindre que les exécutions précédentes mais dans laquelle un gaz liquéfié est dissous (tel que le gaz carbonique, le protoxyde d'azote).

Au moment du déclenchement, l'élément de transmission de pression se déplace très rapidement par la décompression de la substance liquide 7. Quand la pression atteint la pression de changement de phase "liquide-gaz" du gaz liquéfié dissous dans la substance compressible, le gaz prend la relève et continue son expansion tout en propulsant le liquide à injecter à une pression moindre. Cette transformation de phase s'opère à température ambiente aux alentours de 70 bars pour le gaz carbonique.

Dans cette forme d'exécution, le volume du liquide compressible 7 peut être le même que le volume du liquide à injecter 2. Le volume du gaz liquéfié peut être un dixième du volume du liquide compressible 7. Lors de la détente du gaz liquéfié en bulles de gaz 77, la pression reste constante pendant l'écoulement du liquide à injecter 2 à travers l'orifice.

La pointe de pression peut être 5 à 20 fois supérieure à la pression moyenne d'injection. Cette pointe de pression permet de percer facilement le derme et l'épiderme ce qui garantit une injection totale du produit.

Une autre variante d'exécution de la Fig. 35 consiste à inclure avec le liquide compressible 7 un gaz comprimé dans une ou des capsules ou sacs ayant un élément de transmission composé d'une paroi déformable.

Dans cette variante, lors de la décompression, le liquide compressible 7 crée le pic de pression, puis la détente du gaz comprimé contenu dans l'élément de transmission (paroi de polymer) produit une pression plus faible mais néanmoins suffisante pour finir d'injecter le liquide 2.

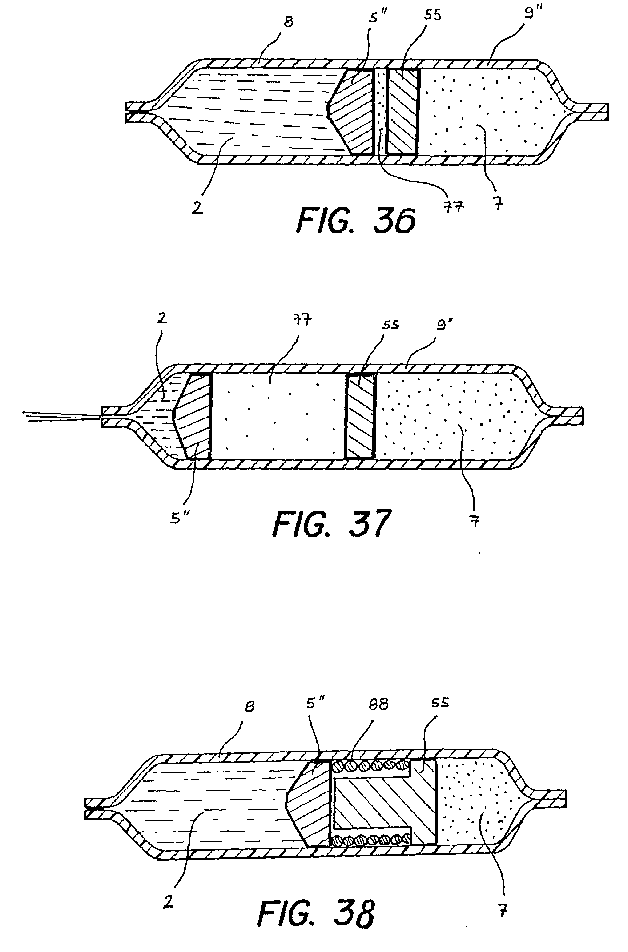

La forme d'exécution de la Fig. 36 consiste à séparer le liquide compressible 7 du gaz liquéfié ou comprimé 77 par un élément de séparation libre comme un piston coulissant 55 dans l'ampoule 4. Le liquide compressible 7 étant comprimé à 500 bars (7% de compressibilité), il fait avancer le piston de 7%, donc 7% du liquide à injecter 2 sera au départ à 500 bars de pression, ce qui perce l'épiderme et le derme, puis le gaz liquéfié ou comprimé prend la relève pour finir d'évacuer le liquide à injecter 2 à une pression de 70 bars pour le gaz carbonique. La Fig. 37 montre la position des pistons 5" et 55 en fin d'injection.

La Fig. 38 montre une variante de la Fig. 36 et la Fig. 37 où le gaz liquéfié ou compressé est remplacé par un ressort (88) comprimé qui peut produire le même effet que le gaz sous pression ou liquéfié.

Dans ces dernières variantes, le système de rétention était représenté par un bouchon de cire au niveau de l'orifice de sortie du liquide, mais il est évident que d'autres dispositifs peuvent être utilisés ou combinés sur la base de ce qui est décrit ci-dessus.

Comme substance liquide compressible, l'on peut également utiliser de l'eau ou des huiles organiques diverses, évidemment au détriment du volume nécessaire pour obtenir le même effet qu'avec les polysiloxanes.

## Revendications

1. Dispositif d'injection hypodermique pour l'administration sous-cutanée, sans aiguille, d'un produit liquide à injecter (2) tel qu'un médicament, un vaccin ou une autre composition pharmaceutique, caractérisé en ce que le dispositif est destiné à l'usage unique et comporte une ampoule contenant ledit produit liquide à injecter et une substance compressible (7, 7') liquide ou solide, une partie de buse (11, 11') ayant un orifice, et des moyens de rétention permettant de maintenir, avant l'utilisation, la substance compressible comprimée à une pression suffisante pour propulser le produit liquide à injecter, pendant l'utilisation, à travers l'orifice de manière à créer un jet de liquide ayant une vitesse suffisante pour traverser la peau d'un patient.

2. Dispositif selon la revendication 1, caractérisé en ce qu'un élément de transmission de pression, tel qu'un piston ou une paroi déformable, disposé entre ces parties, de l'ampoule contenant ledit produit liquide et une partie de l'ampoule contenant la substance compressible.

3. Dispositif selon la revendication 2, caractérisé en ce que l'élément de transmission est un piston (5, 5', 5", 5"', 5"").

4. Dispositif selon la revendication 3, caractérisé en ce que les moyens de rétention comprennent une tige (17, 17', 17", 17"', 17"") retenant le piston (5, 5', 5"") avant l'utilisation dans une position où la substance compressible 7 est comprimée.

5. Dispositif selon la revendication 4, caractérisé en ce que la tige (17, 17"') comporte une zone de rupture (19, 19, 19") fragilisée par une trempe et/ou un enlèvement de matière ou une entaille permettant la libération du piston par la rupture de la tige en cette zone par application d'une force de flexion ou de torsion.

6. Dispositif selon la revendication 4 ou 5, caractérisé en ce que la tige (17, 17"') est fixée de façon permanente, tel que par sertissage ou soudure, à une partie d'extrémité arrière (12, 12''') de l'ampoule.

7. Dispositif selon la revendication 4, caractérisé en ce que la tige (17', 17", 17"") est attachée à l'ampoule par des moyens (26, 27, 28, 29, 30, 31) externes à la tige qui peut être actionnée pour libérer la tige de l'ampoule.

8. Dispositif selon l'une des revendications 4 à 8, caractérisé en ce que le piston (5') comporte une première partie (36) destinée à subir la pression de la substance compressible (7) et une deuxième partie (37) de plus faible section que la première partie destinée à appliquer une pression plus élevée que la pression dans la substance compressible sur le liquide à injecter (2).

9. Dispositif selon l'une des revendications 4 à 8, caractérisé en ce que la tige comporte un passage (32, 33) pour permettre le remplissage de l'ampoule par la substance compressible (7) ou par le liquide à injecter (2).

10. Dispositif selon la revendication 3, caractérisé en ce que le piston (5", 5"') est monté de façon essentiellement flottante dans l'ampoule.

11. Dispositif selon la revendication 2, caractérisé en ce que l'élément de transmission de pression est une paroi déformable (49, 49', 49", 49"").

12. Dispositif selon la revendication 11, caractérisé en ce que la paroi déformable (49, 49') encapsule le liquide à injecter (2).

13. Dispositif selon la revendication 12, caractérisé en ce que la paroi déformable (49') et la partie de buse (11') forment une cartouche stérile contenant le liquide à injecter et qui est montée dans l'ampoule.

14. Dispositif selon la revendication 11, caractérisé en ce que la paroi déformable (49", 49"') encapsule la substance compressible (7, 7').

15. Dispositif selon la revendication 1 ou 2, caractérisé en ce que l'ampoule comporte une partie (8, 8', 8", 8"', 8"") contenant le liquide à injecter (2) et une partie (9, 9', 9", 9"', 9"") contenant la substance compressible.

16. Dispositif selon la revendication 15, caractérisé en ce que la partie (8''') contenant le liquide à injecter est disposée à l'intérieur de la partie (9"') contenant la substance compressible.

17. Dispositif selon la revendication 15, caractérisé en ce que la partie (8, 8', 8") contenant le liquide à injecter est disposée à côté de la partie (9, 9', 9", 9"") contenant la substance compressible.

18. Dispositif selon l'une des revendications 1 à 3 et 10 à 17, caractérisé en ce que les moyens de rétention comportent un bouchon (40, 40', 45, 47, 47') destiné à maintenir la pression de la substance compressible dans l'ampoule avant l'utilisation par la fermeture d'un orifice ou d'un passage (16, 44, 44', 44", 44"').

19. Dispositif selon la revendication 18, caractérisé en ce que le bouchon est disposé dans la partie de buse (11, 11').

20. Dispositif selon la revendication 18, caractérisé en ce que le bouchon est disposé de façon à bloquer un passage (44, 44', 44", 44"') reliant une partie de l'ampoule contenant le liquide à injecter et une partie de l'ampoule contenant la substance compressible.

21. Dispositif selon l'une des revendications 18 à 20, caractérisé en ce que le bouchon (40', 47, 47') est un bouchon mécanique pouvant être déplacé pour libérer lesdits passage ou orifice.

22. Dispositif selon l'une des revendications 18 à 20, caractérisé en ce que le bouchon (44) est en un matériau qui peut être fondu, tel que de la paraffine, ou être décomposé par une sollicitation externe, telle qu'un apport local de chaleur.

23. Dispositif selon une des revendications 18 à 21, caractérisé en ce que le bouchon (47') est attaché à une paroi ou piston amovible (54), disposé dans une partie (9", 9"") de l'ampoule contenant la substance compressible de sorte qu'avant l'utilisation, une petite partie de substance compressible se trouve dans une partie arrière (60') de l'ampoule de façon à maintenir le piston dans une position où le bouchon (47') bloque le passage (44, 44"').

24. Dispositif selon la revendication 23, caractérisé en ce que l'ampoule comporte des moyens pour pouvoir ouvrir la partie arrière (60') de façon à réduire la pression dans cette partie et entraîner le déplacement vers l'arrière du piston (54) et du bouchon (47').

25. Dispositif selon la revendication 24, caractérisé en ce que les moyens d'ouverture de la partie arrière (60') comportent un bouchon arrière (63) muni d'une zone de rupture (66).

26. Dispositif selon la revendication 24, caractérisé en ce que les moyens d'ouverture de la partie arrière (60') comportent une zone de rupture (58) dans la paroi de l'ampoule.

27. Dispositif selon l'une des revendications 23 à 26, caractérisé en ce que le piston amovible comporte un ou plusieurs passages (57) reliant la partie arrière (60') au reste de la partie contenant la substance compressible.

28. Dispositif selon l'une des revendications 1 à 3, 10, 15 ou 16, caractérisé en ce qu'une partie (8"") de l'ampoule contenant le liquide à injecter (2) est sous forme d'une capsule ou cartouche rigide et fermée par rapport à une partie contenant la substance compressible.

29. Dispositif selon la revendication 28, caractérisé en ce que la partie (8"") contenant le liquide à injecter comporte un tube (76) pouvant être brisé pour l'actionnement du dispositif.

30. Dispositif selon la revendication 28, caractérisé en ce que le tube est dimensionné de façon à permettre la création d'une onde de choc résultant de la pression dynamique de la substance compressible dans la partie contenant le liquide à injecter suivant la rupture du tube.

31. Dispositif selon l'une des revendications 28 à 30, caractérisé en ce que la partie contenant le liquide à injecter est en verre.

32. Dispositif selon l'une des revendications précédentes, caractérisé en ce que la substance compressible est mise sous pression dans l'ampoule en réduisant son volume de contenance après l'introduction de la substance compressible.

33. Dispositif selon la revendication 32, caractérisé en ce que le volume de contenance de la substance compressible est réduit par une déformation plastique d'une paroi de l'ampoule.

34. Dispositif selon l'une des revendications précédentes, caractérisé en ce que la partie de l'ampoule contenant la substance compressible comporte une paroi extérieure en métal.

35. Dispositif selon l'une des revendications précédentes, caractérisé en ce que la substance compressible est un liquide viscoélastique.

36. Dispositif selon la revendication 35, caractérisé en ce que la substance compressible est un liquide appartenant à la famille des huiles polysiloxanes.

37. Dispositif selon l'une des revendications 35 ou 36, caractérisé en ce que le dispositif comporte un gas liquéfié dissous dans la substance liquide compressible.

38. Dispositif selon l'une des revendications 35 ou 36, caractérisé en ce que le dispositif comporte un gaz comprimé ou liquéfié contenu dans un élément de transmission de pression contenu dans la même enceinte (9"') que le liquide compressible (7).

39. Dispositif selon la revendication 38, caractérisé en ce que le dispositif comporte un deuxième piston libre (55) coulissant servant à séparer le gaz liquéfié ou comprimé du liquide compressible (7).

40. Dispositif selon la revendication 39, caractérisé en ce que le dispositif comporte à la place du gaz comprimé ou liquéfié un ressort comprimé (88).

41. Dispositif selon l'une des revendications 1 à 27, caractérisé en ce que la substance compressible est un solide élastique.

42. Dispositif selon la revendication 41, caractérisé en ce que le solide est du caoutchouc silicone vulcanisé.
